# EUROPEAN PATENT SPECIFICATION

(11) **EP 2 953 597 B1**
(45) Date of publication and mention of the grant of the patent: **18.03.2020**
(21) Application number: 14748552.8
(22) Date of filing: 07.02.2014
(51) Int. Cl.: A61F 13/00, A61F 13/02, A61F 13/511

(54) **SEE-THROUGH WOUND OR SKIN CONDITION DRESSING AND TREATMENT SYSTEM**
DURCHSICHTIGER WUND- ODER HAUTVERBAND UND BEHANDLUNGSSYSTEM
PANSEMENT TRANSPARENT POUR PLAIE OU AFFECTION CUTANÉE ET SYSTÈME DE TRAITEMENT

(30) Priority: 07.02.2013 US 201361761992 P
(43) Date of publication of application: 16.12.2015
(73) Proprietor: Lagreca, Alfred J., Hingham, MA 02043 (US)
(72) Inventor: Lagreca, Alfred J., Hingham, MA 02043 (US)
(74) Representative: Jones, Graham Henry
(86) International application number: PCT/US2014/015282
(87) International publication number: WO 2014/124242

(56) References cited:
- WO-A1-2011/017384
- WO-A2-94/00090
- US-A- 5 264 218
- US-A1- 2007 161 937
- US-A1- 2009 287 133
- US-A1- 2010 010 458
- US-A1- 2012 116 334
- US-A1- 2012 238 932
- US-B2- 7 576 257

## Description

### Field

This disclosure relates to a dressing for wounds or skin conditions.

### Background

Wound dressings can need to be removed so that the caregiver can check the condition of the wound. The dressing then needs to be replaced. Since the dressings are adhered to the skin, such repeated removal of dressings can damage the skin, cause pain, and lead to infections and other side effects.

### Summary

A purpose of the disclosure is to reduce the number of adverse events caused by the time required for nurses, doctors and patients to remove taped dressings from wounds for evaluation or for changing, as well as to reduce the negative factors related to the removing of the tape. The removal of tape, over the time required for wounds to heal, can cause scaring, infections and dermatitis. Further, the necessary repeated removal is very painful. The 24/7 observation of incisions or wounds without exudates allowed by the transparent cover member can significantly reduce the severity of infections. Also, this wound observation takes place while the wound remains sterile under the transparent cover member. There is thus no additional risk of acquiring a hospital acquired infection (HAI) as can happen when a dressing is lifted or removed so that the wound can be inspected. The see-through nature of the present dressing can lead to a significant reduction in HAIs, which currently cost billions of dollars per year.

A significant cause of HAIs is air-borne pathogens that infect a wound during a dressing change. The present see-through dressing system helps to protect the patient from HAIs, as in many cases because the wound can be inspected under aseptic conditions, the dressing can remain on during the entire hospital stay. Since there are fewer deleterious pathogens in the home environment, changing the dressing at home may reduce the risk of infections.

The disclosure has at least the following benefits:
- Reduces hospital costs by reducing the time it takes a nurse or doctor to change a bandage. This reduces the effectiveness of the medical staff which in turn leads to adverse events such as infections, wrong medications etc. Thousands of patients die each year due to adverse events, most caused by the shortage of medical personnel.
- Reduces disposal of medical waste.
- Increases home care patient independence.
- Reduces unnecessary bandage changes as wound and dressing can be evaluated without removal of bandage.
- May reduce infection from bacteria.
- Reduces pain, scarring and dermatitis caused by the daily changing of a bandage held to the skin with tape.
- Allows bandage to be changed and wound to be evaluated 24/7 or dressed as frequently as desired without irritation that is caused by tape removed from the skin. This allows hospital acquired infections to be identified and treated earlier, which can reduce the seriousness and associated costs.
- Improves the quality of wound management as the type of dressing/medication may be changed without having to remove the base of the bandage.
- Decreases risk of skin conditions such as skin irritations, an example being bed sores, from becoming ulcers. This is because the skin condition may be treated prior an ulcer or wound forming.
- Allows skin tears (which are prevalent in the elderly) to be managed in a sterile, observable environment.

The invention is as defined in the claims.

In US 5,264,218 there is disclosed a wound dressing. The wound dressing is such that, prior to use, it is manufactured to have a central disc which is surrounded by a plurality of rings. Each of the rings is covered by a covering portion which extends over an upper side of the rings, and which is transparent so that the wound can be seen through the covering portion. The rings space the covering portion from the wound. In use, the largest ring is placed around the wound. The covering portion is a semi-permeable thin film which allows moisture/vapour from the wound to pass through the covering. The rate of the moisture/vapour transmission from the wound is controllable by stacking the rings one upon the other. Use of two rings with their covering portions slows the rate of moisture/vapour transmission from the wound by half. If this is not enough, then another ring can be placed on top of the second ring. There is no disclosure of a wound or skin condition dressing and treatment system which, prior to use, is constructed such that there is a single annular base portion, an open area which is defined by the single annular base portion, and which contains a covering portion which extends over an upper side of the annular base portion and which is transparent so that the wound or skin condition can be seen through the covering portion. US 2007/0161937 A1 discloses a wound shield for exudate management.

### Brief Description of the Drawings

Other objects, features and advantages will occur to those skilled in the art from the following description of the preferred embodiments, and the accompanying drawings, in which:
Figure 1 is a cross-sectional view of a two-piece wound dressing system in place surrounding a wound or skin condition;
Figure 2 is a cross-sectional view of a one-piece wound dressing (not part of the invention) system in place surrounding a wound or skin condition; and
Figure 3 is a top view of the one-piece wound dressing (not part of the invention) system shown in Figure 2 and in place surrounding a wound or skin condition.

### Detailed Description of Examples

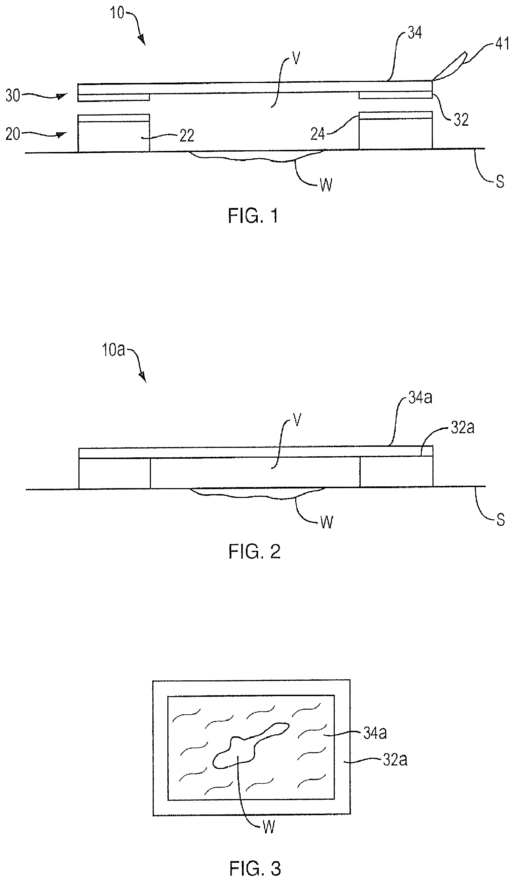

This invention may be accomplished in a disposable or reclosable wound or skin condition dressing and treatment system. The inventive wound or skin condition dressing and treatment system 10, figure 1, has two separable components: a base member 20 that is adhered to skin surface S, and a cover or top member 30 that is removably adhered to the top of base member 20. A bandage, dressing pad or treatment pad itself can be but does not need to be held in place against the skin.

The base member 20 is an annulus or other shape that defines or encompasses an open area. The base member is semi-permanently adhered to the skin, with the wound or skin condition "W" being treated located within the open area such that the base surrounds the wound or skin condition, preferably without touching it. Member 20 can comprise an annulus 22 of a hydrocolloid with an adhesive on the lower side that is covered with a removable protective covering (not shown) that is removed just before use. Top layer 24 of base member 20 can be a polyethylene film (or another plastic from which an adhesive can be released) that is the same size and shape as annulus 22 and is adhered to the top of annulus 22. The base member provides the function of a means to removably couple the top member to the skin, while the top member may be spaced from the skin so as not to disturb the wound.

The adhesion of the base member to the skin may be accomplished with any known adhesive that is compatible with skin. The adhesive may be that used on NEXCARE absolute waterproof first aid tape by the 3M Company, or a hydrocolloid adhesive such as that disclosed in US Patent 4,952,618, for example, or foam tape from 3M. These adhesives may remain in contact with the skin for five to ten days under normal use, including movement and exposure to water, without loosening and without substantial negative impact to the skin. In an embodiment, tape is not used and the base member remains on the skin for multiple days. This obviates the need for frequent removal of tape from the skin that leads to so many problems in the current wound-care regimen.

The base can have a desired, preferably annular shape, such as a circular, oval or rectangular annulus, for example. The adhesive on the lower side of the base is used to secure the base to the person's skin, surrounding the wound or area of the skin requiring covering or treatment. Adhesives that can be used to couple the base to the skin can also include hydrocolloid material such as 3M #9943 or #9944, or Duoderm by Convatec of Skellman, NJ, or an adhesive hydrogel such as available from Scapa North America in Windsor, CT. The adhesive would normally be covered with a removable protective layer as is commonly used to protective the sticky side of an adhesive tape, label or sticker. The base is preferably sterile, and can be made with anti-bacterial material.

Top member 30 can be of the same size and perimeter shape as the base member, except that it is not annular. Accordingly, member 30 covers base member 20, and also spans the opening defined by the base member, to enclose the volume "V" in which wound or skin condition W is located, and also enclose a dressing or treatment pad should such be used. Top member 30 is preferably constructed with an annulus 32 of polyurethane medical grade adhesive tape that overlies some or all of base annulus 22. Top member 30 also includes a semi-permeable sheet or membrane 34. Sheet 34 may be but need not be a polyurethane membrane, one example of which is 3M #9833. Sheet 34 may be secured to annulus 32. Sheet 34 spans the annular opening of base 20. Sheet 34 is preferably adhesive-free so that it does not adhere to the wound or any dressing that touches sheet 34. Top member 30 is sterile and can be made of anti-bacterial material.

Sheet 34 is preferably translucent or clear. Sheet 34 may also be hydrophobic. If sheet 34 is clear or translucent, the pad or the underlying skin region can be inspected without separating the top member from the bottom member.

The subject system is a very low-profile wound covering that can stay in place for many days, thus decreasing the need to pull tape off of the skin in order to view a wound, or change a bandage or treatment pad. The result is less patient pain and suffering, and decreased costs associated with treating infections caused by repeated tape removal, which is prevalent in chronic-care situations in which a dressing may need to be changed multiple times per day, for a week or more. This also decreases medical costs by changing dressings only when necessary, decreased waste and decreased labor costs associated with removing tapes and their residue. Increasing nursing and medical availability caused by this invention will decrease adverse events causing complications and sometimes death. Also, the invention allows for the continuous observation of an incision or wound without exudates. This allows infections, swelling, redness or bursting and other disorders to be identified immediately, which can lead to a significant reduction of the severity of infections and the associated costs.

### Alternative Materials

The subject system contemplates a variety of means for adhesively coupling the base member to the skin of a person or animal, a variety of means for removably coupling the top member to the base member, and a variety of wound and skin condition treatment modalities held in place by the base and top members.

For example, the base can be coupled to the skin with any adhesive that accomplishes sufficient adhesion along with the benefits of less skin irritation or damage. An additional example is adhesive silicone gels such as those manufactured and used by Scapa, Windsor, CT. Also, the inventive dressing can be removably coupled to the skin by an elastic compression wrap bandage overlaying the inventive dressing (such as an Ace bandage from BD, Franklin Lakes, NJ) and wrapped around a portion of the body such as a limb or the torso.

As another example, the top can be coupled to the base by an adhesive on the bottom side of the top member that is sufficient to hold the top on the base but allows the top to be released from the base and removed. The top can then be placed back on the base at least once if desired, and preferably multiple times, over the up to 5-7 day typical useful life of the inventive device. One example of an adhesive that may be useful for this purpose is a medical grade pressure sensitive tape adhesive such as 3M #1510 on one part (e.g., on the (bottom surface of the top member) and an adhesive-releasing plastic surface (e.g., a polyethylene film) on the other part (e.g., making up the top surface of the lower annulus).

There are many examples of substances that can make up or be carried by a pad, gauze or the like that can be held in place by the top member, and lie against the skin, and/or protrude into a wound in the skin. As the inventive system can be opened and reclosed, and allows the top to be replaced, the invention can be use to accomplish a desired treatment regimen for a wound, skin condition, or the like. As one example, the pad can carry or comprise or be entirely made from certain known materials that have known medical effects. Among these are:
- Foam composites such as Mepilex Soft Silicon by MoLnlycke of Denmark. These products typically serve more than purpose, for example a combination which maintains moisture in the wound site, is a barrier to bacteria, and absorbs excess exudates.
- Alginates such as Algicell Calcium Alginate by Derma Sciences of Princeton, NJ. These absorb excess exudates, maintain a moist healing environment, and facilitate autolytic debridement.
- Silver alginate such as Maxsorb Extra by Medline Industries of Mundelsein, IL. In addition to the benefits of the alginate, the silver reduces infections from a wide range of microorganisms.
- Antimicrobial such as Anticoat 7 by Smith and Nephew of London, England. This can be an effective barrier to bacterial penetration that may reduce infections in partial or full thickness wounds.
- Composites such as ThinSite Hydrogel by Swiss America of Waldenburg, Switzerland. These are made of a combination of wound dressings which improve healing, reduce infections and absorb exudates.
- Transparent dressings such as Tegaderm #1628 by 3M allow the wound to be viewed at all times but have the disadvantage of difficulty in removal from the skin and wound. The invention significantly reduces the damage of removal but allows for continued viewing of the wound or skin condition or the like.
- Hydrocolloids such as Dermatell by Gentell, Inc of Skellman, NJ. These support a moist healing environment and autolytic debridement and non traumatic dressing removal.
- Hydrogels such as Ameriderm wound gel dressing by Ameriderm Labs Ltd. of Patterson, NJ. The gel's high glycerin content facilitates the natural wound healing process.
- Silver such as Silverlon by Argentum Medical LLC of Willowbrook, Illinois reduces infections by resisting and eliminating most microorganisms.

Of course, the invention contemplates other materials, medications and the like, to accomplish any desired or custom treatment of a condition such as skin irritation caused by pressure prior to the wound developing, or a surface wound. The invention speeds and eases wound and skin care and treatment in situations in which the skin needs to be covered. The invention also decreases incidence of tape removal-related infections. The invention thus can reduce the costs associated with wound and skin care.

The single layer dressing 10a, figures 2 and 3, is
essentially top member 30 from figure 1. Adhesive annulus 32a carries transparent sheet 34a, which spans the open area of the annulus. Annulus 32a can be placed directly on the skin. The result is a clear window through which the wound, or any dressing or the like held in place over the wound, can be continuously viewed.

## Claims

1. A disposable or re-closable wound or skin condition dressing and treatment system (10), comprising
(i) a single annular base portion (20);
(ii) an open area (V) which is defined by the annular base portion (20), and which is for surrounding the wound or skin condition (W);
(iii) a first adhesive which is of annular form, which is on a lower side of the annular base portion (20), and which enables the annular base portion (20) to adhere to the skin;
(iv) a removable protective cover over the first adhesive;
(v) a covering portion (30) which is sterile, which includes a translucent semi-permeable sheet or membrane (34), which extends over an upper side of the annual base portion (20), which fully covers the open area defined by the annular base portion (20), which allows for continued viewing of the wound or skin condition (W) through the covering portion (30), and which is spaced from the wound or skin condition (W); and
(vi) a second adhesive (32) and a top layer (24), and wherein
the second adhesive (32) is of annular form, the second adhesive (32) is on a lower side of the covering portion (30), the top layer (24) is of annular form, the top layer (24) is on the upper side of the annular base portion (20), and the second adhesive (32) releasably engages the top layer (24) whereby the covering portion (30) is a re-closable covering portion (30).

2. A disposable or re-closable wound or skin condition dressing and treatment system (10) according to claim 1 in which the covering portion (30) defines a pull tab which is adapted to be gripped by a user to assist in separating the covering portion (30) from the annular base portion (20).

3. A disposable or re-closable wound or skin condition dressing and treatment system (10) according to claim 1 or claim 2 in which the annular base portion (20) is of a circular, oval or rectangular shape.

4. A disposable or re-closable wound or skin condition dressing and treatment system (10) according to any one of the preceding claims in which the first adhesive is a hydrocolloid adhesive.

## Patentansprüche

1. Einweg- oder wiederschließbares Wund- oder Hautkrankheitsauflage- und -behandlungssystem (10), umfassend:
(i) einen einzelnen ringförmigen Basisabschnitt (20),
(ii) einen offenen Bereich (V), der durch den ringförmigen Basisabschnitt (20) definiert ist und der für das Umgeben der Wunde oder der Hautkrankheit (W) bestimmt ist,
(iii) einen ersten Klebstoff, der ringförmig ist und der sich auf einer unteren Seite des ringförmigen Basisabschnitts (20) befindet und dank dessen der ringförmige Basisabschnitt (20) an der Haut haften kann,
(iv) eine entfernbare Schutzabdeckung über dem ersten Klebstoff,
(v) einen Abdeckabschnitt (30), der steril ist und der eine durchscheinende, halbdurchlässige Folie oder Membran (34) aufweist, die sich über eine obere Seite des ringförmigen Basisabschnitts (20) erstreckt und den durch den ringförmigen Basisabschnitt (20) definierten offenen Bereich vollständig abdeckt, die fortgesetztes Einsehen der Wunde oder der Hautkrankheit (W) durch den Abdeckabschnitt (30) gestattet und die von der Wunde oder der Hautkrankheit (W) beabstandet ist, und
(vi) einen zweiten Klebstoff (32) und eine obere Lage (24), und wobei der zweite Klebstoff (32) ringförmig ist, sich der zweite Klebstoff (32) auf einer unteren Seite des Abdeckabschnitts (30) befindet, die obere Lage (24) ringförmig ist, sich die obere Lage (24) auf der oberen Seite des ringförmigen Basisabschnitts (20) befindet und der zweite Klebstoff (32) die obere Lage (24) lösbar in Eingriff nimmt, wodurch der Abdeckabschnitt (30) ein wiederverschließbarer Abdeckabschnitt (30) ist.

2. Einweg- oder wiederschließbares Wund- oder Hautkrankheitsauflage- und -behandlungssystem (10) nach Anspruch 1, wobei der Abdeckabschnitt (30) eine Zuglasche definiert, die dazu ausgeführt ist, von einem Benutzer ergriffen zu werden, um zum Trennen des Abdeckabschnitts (30) von dem ringförmigen Basisabschnitt (20) beizutragen.

3. Einweg- oder wiederschließbares Wund- oder Hautkrankheitsauflage- und -behandlungssystem (10) nach Anspruch 1 oder Anspruch 2, wobei der ringförmige Basisabschnitt (20) kreisförmig, oval oder rechteckig ist.

4. Einweg- oder wiederschließbares Wund- oder Hautkrankheitsauflage- und -behandlungssystem (10) nach einem der vorhergehenden Ansprüche, wobei der erste Klebstoff ein Hydrokolloid-Klebstoff ist.

## Revendications

1. Système (10) de traitement et de pansement jetable ou refermable pour plaie ou affection cutanée, comprenant :
(i) une portion de base annulaire unique (20) ;
(ii) une zone ouverte (V) qui est définie par la portion de base annulaire (20) et qui est prévue pour entourer la plaie ou l'affection cutanée (W) ;
(iii) un premier adhésif qui présente une forme annulaire, qui est situé sur un côté inférieur de la portion de base annulaire (20) et qui permet à la portion de base annulaire (20) d'adhérer à la peau ;
(iv) un revêtement protecteur amovible par-dessus le premier adhésif ;
(v) une portion de recouvrement (30) qui est stérile, qui comporte une feuille ou membrane semi-perméable translucide (34), qui s'étend par-dessus un côté supérieur de la portion de base annulaire (20), qui recouvre complètement la zone ouverte définie par la portion de base annulaire (20), qui permet de voir en continu la plaie ou l'affection cutanée (W) à travers la portion de recouvrement (30) et qui est espacée de la plaie ou de l'affection cutanée (W) ; et
(vi) un deuxième adhésif (32) et une couche supérieure (24), et dans lequel
le deuxième adhésif (32) présente une forme annulaire, le deuxième adhésif (32) est disposé sur un côté inférieur de la portion de recouvrement (30), la couche supérieure (24) présente une forme annulaire, la couche supérieure (24) est disposée sur le côté supérieur de la portion de base annulaire (20), et le deuxième adhésif (32) vient en prise de manière détachable avec la couche supérieure (24), la portion de recouvrement (30) étant une portion de recouvrement refermable (30).

2. Système (10) de traitement et de pansement jetable ou refermable pour plaie ou affection cutanée selon la revendication 1, dans lequel la portion de recouvrement (30) définit une languette de traction qui est prévue pour être saisie par un utilisateur pour faciliter la séparation entre la portion de recouvrement (30) et la portion de base annulaire (20).

3. Système (10) de traitement et de pansement jetable ou refermable pour plaie ou affection cutanée selon la revendication 1 ou la revendication 2, dans lequel la portion de base annulaire (20) présente une forme circulaire, ovale ou rectangulaire.

4. Système (10) de traitement et de pansement jetable ou refermable pour plaie ou affection cutanée selon l'une quelconque des revendications précédentes, dans lequel le premier adhésif est un adhésif hydrocolloïde.
